# EUROPÄISCHE PATENTSCHRIFT

(11) **EP 0 496 950 B1**
(45) Veröffentlichungstag und Bekanntmachung des Hinweises auf die Patenterteilung: **04.10.1995**
(21) Anmeldenummer: 91117949.7
(22) Anmeldetag: 22.10.1991
(51) Int. Cl.: A61B 17/16, A61B 17/17

(54) **Zielgerät für ein Implantat zur Versorgung trochanterer und subtrochanterer Frakturen**
Implant guide tool for the treatment of trochanter and subtrochanter fractures
Dispositif de visée pour un implant de traitement de fractures trochanterienne et substrochanterienne

(30) Priorität: 30.01.1991 DE 9101037 U
(43) Veröffentlichungstag der Anmeldung: 05.08.1992
(73) Patentinhaber: Howmedica GmbH, D-24232 Schönkirchen (DE)
(72) Erfinder: Behrens, Klaus Friedrich Adolf, Dipl.-Ing., W-2351 Rickling (DE)
(74) Vertreter: Patentanwälte Hauck, Graalfs, Wehnert, Döring, Siemons

(56) Entgegenhaltungen:
- EP-A- 0 273 872
- US-A- 4 622 959
- US-A- 4 733 654

## Beschreibung

Die Erfindung bezieht sich auf ein Zielgerät für ein Implantat zur Versorgung trochanterer und subtrochanterer Frakturen nach dem Oberbegriff des Patentanspruchs 1.

Aus der US-A-4 622 959 ist ein Zielgerät bekanntgeworden, das an das proximale Ende eines in einen Femurkanal eingesetzten Femurnagels anschraubbar ist. Bei einer Ausführungsform weist ein sich annähernd parallel zum Femurnagel erstreckender Zielarm des Zielgerätes mehrere axial beabstandete Querzielbohrungen auf zur Aufnahme von Zielhülsen. Dadurch ist es möglich, eine Anpassung an Femurnägel unterschiedlicher Länge vorzunehmen, bei denen die Querbohrungen axial an unterschiedlichen Positionen vorgesehen sind. Bei einer anderen Ausführungsform des Zielgerätes sind zwei Schrägzielbohrungen im Arm des Zielgerätes vorgesehen, die mit Schrägbohrungen des Femurnagels im Bereich des Femurhalses ausgerichtet sind. Dadurch ist es möglich, Bohrungen durch den Femurhals zu führen und anschließend Schenkelhalsschrauben durch die Schrägbohrungen im Femurnagel einzuschrauben.

Aus der US-A-4 733 654 ist ein Zielgerät bekanntgeworden, das einen rohrartigen am unteren Ende geschlitzten Zapfen aufweist, der in den hohlen oberen Ansatz eines Femurnagels einsteckbar ist. Ein sich parallel zum Femurnagel erstreckender Zielarm weist zwei axial beabstandete Schrägzielbohrungen auf, deren Achsen parallel verlaufen. Außerdem sind unterhalb der Schrägzielbohrungen zwei Querzielbohrungen vorgesehen, die mit Querbohrungen des Femurnagels ausgerichtet sind, wobei die Achsen ebenfalls parallel verlaufen. Bei Femurnägeln, deren Schrägbohrungen unterschiedlich geneigte Winkel aufweisen, ist jeweils ein separates Zielgerät zu verwenden.

Aus der EP-A-0 273 872 ist schließlich bekanntgeworden, eine Führungsvorrichtung zum Bohren von Löchern in den proximalen Femur vorzusehen mit unterschiedlich geneigten Schrägzielbohrungen zur Aufnahme entsprechender Zielhülsen. Auch bei diesem Gerät muß eine andere Ausführungsform gewählt werden, wenn die Neigung der Schrägbohrungen im Femurnagel eine andere ist.

Der Erfindung liegt die Aufgabe zugrunde, ein Zielgerät für ein Implantat zur Versorgung trochanterer und subtrochanterer Frakturen zu schaffen, das als Eintreibwerkzeug verwendbar ist und bei Femurnägeln mit unterschiedlich geneigten Schrägbohrungen verwendet werden kann.

Diese Aufgabe wird durch die Merkmale des Patentanspruchs 1 gelöst.

Die unlösbare Verbindung des Zielarms mit dem Kopf bedingt eine starre Konstruktion, die ein einwandfreies Ausrichten der Ziel- bzw. Bohrhülsen auf die Schräg- und Querbohrungen ermöglicht und damit auch ein genaues Plazieren von Schenkelhals- bzw. Verriegelungsschrauben in Verbindung mit dem Femurnagel. Außerdem kann bei dieser Ausführung des Zielgerätes dieses auch als Werkzeug eingesetzt werden, um den Nagel mehr oder weniger weit in den Femur von proximal einzutreiben.

In Anpassung an die jeweilige Anatomie können die Schrägbohrungen in den Femurnägeln unterschiedliche Winkel aufweisen. Das erfindungsgemäße Gerät besitzt einen Satz von Schrägzielbohrungen, deren Achse und deren Winkel an Achse und Winkel der Schrägbohrungen im Femurnagel angepaßt sind. Für einen bestimmten Satz von Femurnägeln mit unterschiedlichen Schrägbohrungen braucht daher nur ein einziges Zielgerät mit einem entsprechenden Satz von Schrägzielbohrungen vorgesehen werden. Das Aussuchen und die Montage verschiedener Zielarme entfällt, wodurch die Handhabung des Zielgeräts erheblich vereinfacht ist und zu weniger Fehlern führt.

Nach einer Ausgestaltung der Erfindung schließen die Zielbohrungen für verschieden ausgerichtete Schrägbohrungen Winkel von 125°, 130°, 135° und 140° mit der Zielarmachse ein. Diese Auswahl trägt den üblichen anatomischen Bedingungen Rechnung.

Nach einem weiteren Merkmal der Erfindung wird ein Teil der Schrägzielbohrungen von der Querzielbohrung gekreuzt. Dadurch lassen sich alle Zielbohrungen in einem relativ kurzen Abschnitt des Zielgerätes unterbringen, so daß das Zielgerät außerordentlich kompakt ausgeführt werden kann.

Gemäß einer Ausgestaltung hat der Zielarm einen vom freien Ende ausgehenden und die Zielbohrungen achsparallel kreuzenden Klemmschlitz, der ein Festlegen der Bohrhülsen (zur Aufnahme eines Spezialbohrers für die Schenkelhals- und Verriegelungsschrauben) ermöglicht. Die Bohrhülsen sollen leichtgängig in die Zielbohrungen einführbar sein, so daß die durch den Klemmschlitz getrennten Querschnittsbereiche des Zielarms zum Festlegen der Bohrhülse in der Regel gegeneinander verspannt werden müssen. Bevorzugt erstreckt sich dabei der Klemmschlitz nur über einen Teil des Zielarmquerschnitts, so daß ein leichtgängiges Einführen der Bohrhülse nicht durch eine übermäßige Flexibilität des Zielarmes im Bohrungsbereich beeinträchtigt ist.

Gemäß einer bevorzugten Ausgestaltung weist eine auf den Zielarm im Bereich der Zielbohrungen steckbare Hülse einen ersten Wandabschnitt auf, der unter Federvorspannung mit einem zweiten Wandabschnitt verriegelbar ist, wobei der erste Wandabschnitt durch den Klemmschlitz getrennte Bereiche des Zielarmquerschnitts aufeinander zu drückt, und die Hülse mit den Zielbohrungen korrespondierende Durchführbohrungen aufweist. Die Hülse wird über das freie Ende des Zielarms aufgeschoben, worauf die Bohrhülse durch die Durchführbohrungen und Zielbohrungen geführt werden kann. Schließlich wird der Zielarmquerschnitt durch Verriegeln der beiden Wandabschnitte miteinander im Bereich des Klemmschlitzes zusammengedrückt, wodurch die Bohrhülse am Zielarm festgelegt wird.

Bei einer praktischen Weiterbildung hat der Zielarm einen etwa rechtwinkligen Querschnitt und die Hülse einen korrespondierenden Kastenquerschnitt, sind der erste und der zweite Wandabschnitt zwei senkrecht aufeinander ausgerichtete Hülsenwände, und ist der erste Wandabschnitt ausgehend von seiner fest mit einer weiteren Hülsenwand verbundenen Seite vom Zentrum der Hülse weg gebogen und mit seinem freien Rand hinter einem Vorsprung der von ihm getrennten Hülsenwand des zweiten Wandabschnitts verrastbar. Diese Hülse ist preisgünstig aus einem federelastischen Kunststoff oder Metall herstellbar.

Gemäß einer anderen Weiterbildung sind mehrere auf den Zielarm im Bereich der Zielbohrungen steckbare und an diesem festlegbare Hülsen vorgesehen, die jeweils nur eine Durchführbohrung für eine der verschiedenen Zielbohrungen des Zielarmes für Schrägbohrungen und ggf. mindestens eine Durchführbohrung für eine Zielbohrung des Zielarms für eine Querbohrung haben. Je nach ausgewähltem Femurnagel muß entsprechend der Ausrichtung seiner Schrägbohrung eine Hülse ausgewählt werden, die über ihre Durchführbohrungen nur ein Einführen der Bohrhülse in die korrespondierende Zielbohrung ermöglicht.

Ferner sieht eine bevorzugte Weiterbildung vor, daß der Kopf und der Zielarm über einen durchstrahlbaren Haltebogen unlösbar miteinander verbunden sind, wodurch eine Positionsanalyse des Nagels und der Schenkelhalsschraube gegeben wird. Als durchstrahlbares Material kommt ein Kohlenstoffaser- oder ein ähnlicher Kunststoff in Betracht. Vorteilhafterweise bestehen Kopf und Haltearm aus Leichtmetall, vorzugsweise aus einer Aluminium- oder Titanlegierung, so daß die Leichtigkeit des Zielgerätes seine Handhabung begünstigt.

Schließlich sieht eine Ausgestaltung vor, daß der Kopf etwa in der von Zapfenachse und Zielarm aufgespannten Ebene zwei etwa parallele und ungefähr senkrecht zur Zapfenachse ausgerichtete Führungsbohrungen zur Aufnahme seitlich überstehender Kirschner-Drähte für eine Röntgen-Positionskontrolle hat. Kommen die Kirschner-Drähte auf der Röntgenaufnahme (in lateral-medialer Richtung) zur Deckung, ist sichergestellt, daß die Aufnahmeachse in der Ebene von Femurnagel und Zielarm liegt.

Weitere Einzelheiten und Vorteile des Gegenstandes der Neuerung ergeben sich aus der nachfolgenden Beschreibung der zugehörigen Zeichnungen, die eine bevorzugte Ausführungsform des neuerungsgemäßen Gerätes zeigen. In den Zeichnungen zeigen:
- Fig. 1: ein Zielgerät in Seitenansicht;
- Fig. 2: ein Zielarm desselben Zielgerätes mit aufgeschobener Hülse von unten;
- Fig. 3: dasselbe Zielgerät beim Eindrehen einer Schenkelhalsschraube in den gesetzten Femurnagel in stark vereinfachender Seitenansicht.

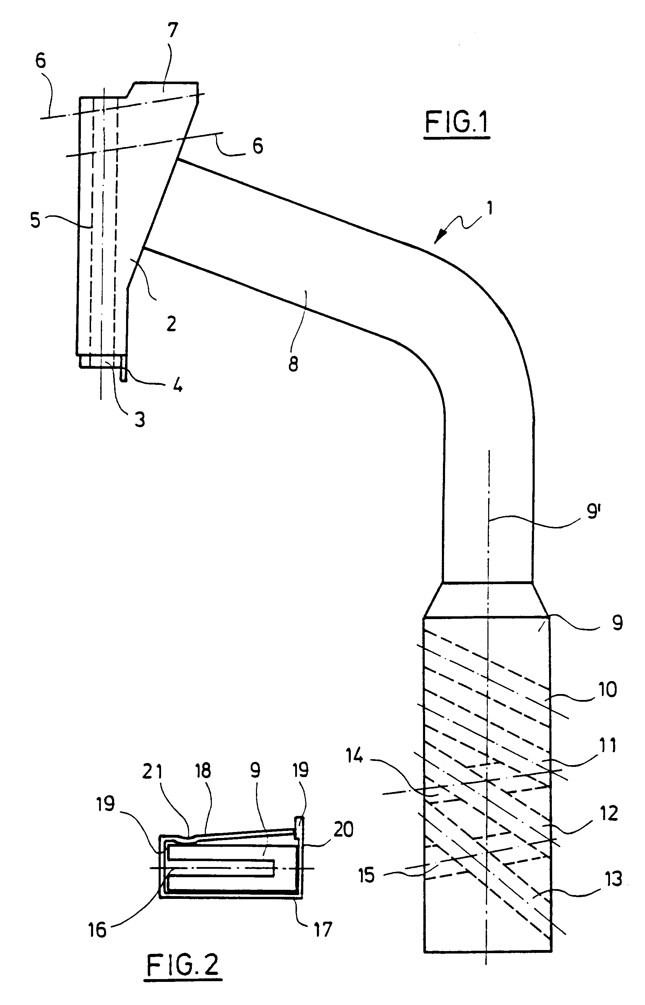

Gemäß Fig. 1 hat das Zielgerät 1 einen Kopf 2, der unterseitig einen Zapfen 3 zum Aufschieben eines Femurnagels aufweist. Neben dem Zapfen 3 ist eine Nase 4 für eine Drehverriegelung in einer entsprechenden Aufnahme des Femurnagels vorgesehen. Der Kopf 2 hat eine Bohrung 5, durch die von oben eine Arretierungsschraube zum Fixieren des Femurnagels am Zapfen 3 eindrehbar ist.

Im Oberbereich des Kopfes 2 befinden sich zwei parallele Führungsbohrungen 6 mit relativ geringem Durchmesser für die Aufnahme von Kirschner-Drähten, die in der Zeichenebene verlaufen. Die oberste Führungsbohrung 6 schneidet zum Teil einen Ansatz 7 für ein Schlagwerkzeug.

Der Kopf 2 ist über einen Haltebogen 8 aus durchstrahlbarem Material unlösbar mit einem Zielarm 9 verbunden. Die Längsachse 9' des Zielarms 9 ist etwa parallel zur Achse des Zapfens 3 und somit zu einem daran befestigbaren Femurnagel ausgerichtet.

Im Zielarm 9 sind erste Zielbohrungen 10, 11, 12, 13 angeordnet, die verschiedene Winkel mit der Zielarmachse 9' von 125°, 130°, 135° und 140° einschließen. Sie dienen der Aufnahme einer Bohrhülse für ein Bohrwerkzeug, wobei die jeweilige Zielbohrung 10, 11, 12, 13 entsprechend der Ausrichtung der Schrägbohrung im nach anatomischen Gegebenheiten ausgewählten Femurnagel gewählt wird.

Der Zielarm 9 hat ferner zweite Zielbohrungen 14, 15 zur Aufnahme der Bohrhülse und Ausrichten derselben auf distale Querbohrungen des Femurnagels. In der Regel hat jeder Femurnagel zwei distale Querbohrungen, die bei sämtlichen Nageltypen an der gleichen Stelle positioniert sind. Im unteren Bereich des Zielarms 9 kreuzen sich Zielbohrungen 11, 12 für Schrägbohrungen mit Zielbohrungen 14, 15 für Querbohrungen des Femurnagels.

Wie der Fig. 2 entnehmbar ist, hat der Zielarm 9 einen vom freien Ende ausgehenden Längsschlitz 16, der sich entlang der dem zu befestigenden Femurnagel zugewandten Seite des Zielarms erstreckt. Die dem Klemmschlitz benachbarten zungenförmigen Querschnittsbereiche des Zielarms 9 lassen sich elastisch zusammendrücken, womit eine Fixierung einer Bohrhülse in den Zielbohrungen 10 bis 15 erreicht werden kann.

Hierzu ist auf den Zielarm 9 eine Hülse 17 mit Kastenprofil aufgeschoben, deren obere Hülsenwand 18 nahe der längsgeschlitzten Seite des Zielarms 9 an eine seitliche Hülsenwand 19 einteilig angebunden ist. Der andere Rand der Hülsenwand 18 ist frei und im entspannten Zustand etwas vom Zielarm 9 weggebogen. Er ist unter Federvorspannung unter einen Vorsprung 19 der anderen seitlichen Hülsenwand 20 drückbar und hierdurch mit dieser verriegelbar. Im verriegelten Zustand drückt eine parallel zur Zielachse verlaufende Eindellung 21 den Zielarm im Bereich des Klemmschlitzes 16 zusammen, wodurch die Bohrhülse festgelegt ist. Die seitlichen Hülsenwände 19, 20 haben - nicht dargestellt - Durchführbohrungen für eine Bohrhülse.

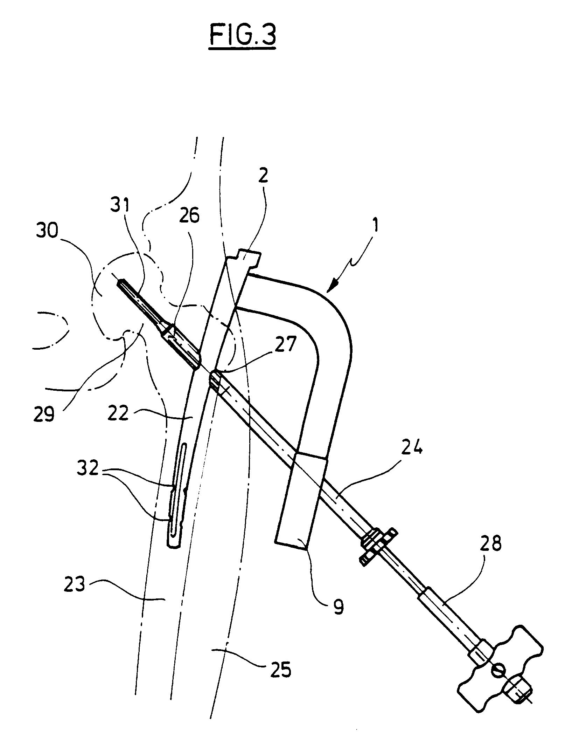

Die Fig. 3 zeigt das Zielgerät 1 mit einem am Kopf 2 festgelegten Femurnagel 22, der bereits in den Markraum eines Oberschenkelknochens 23 eingedrückt ist. Eine schon bekannte Bohrhülse 24 ist in eine Zielbohrung des Zielarms 9 eingeschoben und darin fixiert. Die Bohrhülse 24 ist vorn in die Weichteile 25 und den Oberschenkelknochen 23 gedrückt, wozu sie am vorderen Ende Zacken aufweisen kann, die auch eine Fixierung am Knochen selbst ermöglichen. Eine Oberschenkelhalsschraube 26 ist durch die Bohrhülse 24 in eine Schrägbohrung 27 des Femurnagels geschoben und wird mittels eines Spezialschraubers 28 in Schenkelhals 29 und Femurkopf 30 eingedreht, wozu sie vorn ein Gewinde 31 aufweist. In der gezeigten Lage kann die Oberschenkelhalsschraube 26 mittels einer - nicht dargestellten - Sicherungsschraube axial verschieblich, jedoch unverdrehbar im Femurnagel 22 gesichert werden.

Falls erforderlich, wird die Bohrhülse 24 in einem weiteren Operationsschritt in eine der Zielbohrungen eingeschoben, die auf die distalen Querbohrungen 32 des Femurnagels 22 ausgerichtet sind. Dafür muß ggf. die Klemmeinrichtung einer auf den Zielarm 9 aufgeschobenen Hülse 17 (vgl. Fig. 2) gelockert werden, damit die Bohrhülse 24 von einer Zielbohrung in die andere gebracht werden kann.

## Patentansprüche

1. Zielgerät für ein Implantat zur Versorgung trochanterer und subtrochanterer Frakturen mit einem Kopf (2), der einen Zapfen (3) zum Halten eines in den Markraum einführbaren Femurnagel (22) und mit einem seitlich am Kopf (2) befestigten Zielarm (9), der sich etwa parallel zum Femurnagel (22) erstreckt und mindestens zwei auf proximale Schrägbohrungen (27) des Femurnagels (22) für eine Schenkelhalsschraube (26) ausgerichtete Schrägzielbohrungen (10 bis 13) und mindestens eine auf eine distale Querbohrung (32) des Femurnagels (22) für eine distale Verriegelungsschraube ausgerichtete Querzielbohrung (14, 15) zur Aufnahme einer Bohrhülse (24) aufweist, dadurch gekennzeichnet, daß der Kopf unlösbar am Zielarm (9) befestigt ist und Arretiermittel zur lösbaren Befestigung sowie eine Schlagfläche (7) zum Eintreiben des Femurnagels (2) aufweist und daß der Zielarm (9) mehrere Schrägzielbohrungen (10 bis 13) aufweist, die unterschiedliche Winkel mit der Zielarmachse (9') einschließen und zumindest zum Teil von der Querzielbohrung (14, 15) gekreuzt werden.

2. Zielgerät nach Anspruch 1, dadurch gekennzeichnet, daß die Schrägzielbohrungen (10 bis 13) für verschieden ausgerichtete Schrägbohrungen (27) Winkel von 125°, 130°, 135° und 140° mit der Zielarmachse (9') einschließen.

3. Zielgerät nach Anspruch 1 oder 2, dadurch gekennzeichnet, daß der Zielarm (9) einen vom freien Ende ausgehenden und die Zielbohrungen achsparallel kreuzenden Klemmschlitz (16) hat.

4. Zielgerät nach Anspruch 3, dadurch gekennzeichnet, daß sich der Klemmschlitz (16) nur über einen Teil des Zielarmquerschnitts erstreckt.

5. Kombination eines Zielgeräts nach Anspruch 4 mit einer Hülse, dadurch gekennzeichnet, daß eine auf den Zielarm (9) im Bereich der Zielbohrungen (10 bis 15) steckbare Hülse (17) einen ersten Wandabschnitt (18) aufweist, der unter Federvorspannung mit einem zweiten Wandabschnitt (20) verriegelbar ist, wobei der erste Wandabschnitt durch den Klemmschlitz (16) getrennte Bereiche des Zielarmquerschnitts aufeinander zu drückt, und daß die Hülse (17) mit den Zielbohrungen (10 bis 15) korrespondierende Durchführbohrungen aufweist.

6. Kombination nach Anspruch 5, dadurch gekennzeichnet, daß der Zielarm (9) einen etwa rechtwinkligen Querschnitt und die Hülse (17) einen korrespondierenden Kastenquerschnitt haben, daß der erste und der zweite Wandabschnitt zwei senkrecht aufeinander ausgerichtete Hülsenwände (18, 20) sind, daß der erste Wandabschnitt (18) ausgehend von seiner fest mit einer weiteren Hülsenwand (19) verbundenen Seite vom Zentrum der Hülse (17) weg gebogen ist und mit seinem freien Rand unter einen Vorsprung (19) der von ihm getrennten Hülsenwand (20) des zweiten Wandabschnitts verrastbar ist.

7. Kombination nach einem der Ansprüche 6 oder 7, dadurch gekennzeichnet, daß mehrere auf den Zielarm (9) im Bereich der Zielbohrungen (10 bis 15) steckbare und an diesem festlegbare Hülsen (17) vorgesehen sind, die jeweils nur eine Durchführbohrung für eine der verschiedenen Zielbohrungen (10 bis 13) des Zielarms für Schrägbohrungen (27) und ggf. mindestens eine Durchführbohrung für eine Zielbohrung (14, 15) des Zielarms für eine Querbohrung (32) haben.

8. Zielgerät nach einem der Ansprüche 1 bis 4, dadurch gekennzeichnet, daß der Kopf (2) und der Zielarm (9) über einen durchstrahlbaren Haltebogen (8) unlösbar miteinander verbunden sind.

9. Zielgerät nach einem der Ansprüche 1 bis 4 oder 8, dadurch gekennzeichnet, daß der Kopf (2) und der Zielarm (9) aus Leichtmetall, vorzugsweise eine Aluminium- oder Titanlegierung bestehen.

10. Zielgerät nach einem der Ansprüche 1 bis 4, 8 oder 9, dadurch gekennzeichnet, daß der Kopf etwa in der von Achse des Zapfens (3) und Zielarm (9) aufgespannten Ebene zwei etwa parallele und ungefähr senkrecht zur Zapfenachse ausgerichtete Führungsbohrungen (6) zur Aufnahme seitlich überstehender Kirschner-Drähte für eine Röntgen-Positionskontrolle hat.

## Claims

1. An implant guide tool for the treatment of trochanter and subtrochanter fractures having a head (2) which comprises a pin (3) for holding a femur nail (22) adapted to be inserted into the medullary canal, and a guide arm (9) secured to said head (2) at the side, which guide tool (9) extends approximately parallel to said femur nail (22) and includes at least two inclined target bores (10 to 13) aligned to inclined proximal bores (27) of said femur nail (22) for a femoral neck screw (26) and at least one cross target bore (14, 15) aligned to a distal cross bore (32) of said femur nail (22) for a distal locking screw for receiving a drill sleeve (24), characterized in that said head is nondetachably secured to said guide arm (9) and comprises locking means for a detachable fastening as well as an impact surface (7) for driving in said femur nail (22), and that said guide arm (9) comprises several inclined target bores (10 to 13) which include different angles with the guide arm axis (9') and are at least partly crossed by said cross target bore (14, 15).

2. The guide tool according to claim 1, characterized in that said inclined target bores (10 to 13) for differently aligned inclined bores (27) include angles of 125°, 130°, 135° and 140° with said guide arm axis (9').

3. The guide tool according to claim 1 or 2, characterized in that said guide arm (9) comprises a clamping slot (16) starting from the free end and crossing said target bores parallel to the axis.

4. The guide tool according to claim 1, characterized in that said clamping slot (16) only extends over part of the cross section of said guide arm.

5. Combination of a guide tool according to claim 4, characterized in that a bush (17) to be put on said guide arm (9) within the area of said target bores (10 to 15) comprises a first wall portion (18) which is lockable with a second wall portion (20), with the spring being pretensioned, said first wall portion forcing towards each other portions of the cross section of said guide arm separated from each other by said clamping slot (16), and that said bush (17) comprises lead-through bores corresponding to said target bores (10 to 15).

6. Combination according to claim 5, characterized in that said guide arm (9) has an approximate rectangular cross section and said bush (17) has a corresponding box section, that said first and said second wall portions are two bush walls (18, 20) perpendicularly aligned to each other, that said first wall portion (18), starting from its side fixedly connected to another bush wall (19), is bent away from the centre of said bush (17) and with its free edge can lock into place under a projection (19) of said bush wall (20) of said second wall portion separated therefrom.

7. Combination according to any of the claims 6 or 7, characterized in, that there are provided several bushes (17) adapted to be put on and fixed to said guide arm (9) within the area of said target bores (10 to 15), each of which bushes (17) comprises only one lead-through bore for one of the various target bores (10 to 13) of said guide arm for inclined bores (27) and, if necessary, at least one lead-through bore for a target bore (14, 15) of said guide arm for a cross bore (32).

8. The guide tool according to any of the claims 1 to 4, characterized in that said head (2) and said guide arm (9) are nondetectably connected to each other by means of a supporting bow (8) being X-ray transparent.

9. The guide tool according to any of the claims 1 to 4 or 8, characterized in that said head (2) and said guide arm (9) are composed of light metal, preferably an aluminium or titanium alloy.

10. The guide tool according to any of the claims 1 to 4, 8 or 9, characterized in that, approximately in the plane resulting from the axis of said pin (3) and said guide arm (9), said head comprises two approximately parallel guide bores (6) aligned approximately perpendicular to the axis of said pin for receiving Kirchner wires for a X-ray position control, said Kirchner wires projecting at the side.

## Revendications

1. Dispositif de visée pour un implant de traitement de fractures trochantériennes et sous-trochantériennes, avec une tête (2) présentant un tenon (3) pour fixer une broche fémorale (22) qui peut être introduit dans la cavité médullaire et avec un bras de visée (9) fixé latéralement à la tête (2) qui s'étend à peu près parallèlement à la broche fémorale (22), et au moins deux trous de visée obliques (10 à 13) alignés sur des trous obliques proximaux (27) de la broche fémorale (22) prévus pour une vis de col de fémur (26), et au moins un trou de visée transversal (14, 15) aligné sur un trou transversal distal (32) de la broche fémorale (22) pour une vis de verrouillage distale et destiné à recevoir un tube de forage (24),
caractérisé en ce que la tête est fixée de manière indissociable au bras de visée (9) et présente des moyens d'immobilisation pour une fixation amovible ainsi qu'une surface de frappe (7) pour l'enfoncement de la broche fémorale,
et en ce que le bras de visée (9) comporte plusieurs trous de visée obliques (10 à 13) formant un angle différent avec l'axe de bras de visée (9') et recoupés au moins partiellement par le trou de visée transversal (14, 15).

2. Dispositif selon la revendication 1, caractérisé en ce que, pour des trous obliques (27) orientés différemment, les trous de visée obliques (10 à 13) forment, avec l'axe (9') du bras de visée, des angles de 125°, 130°, 135° et 140°.

3. Dispositif de visée selon la revendication 1 ou 2, caractérisé en ce que le bras de visée (9) présente une fente de serrage (16) partant de son extrémité libre et croisant les trous de visée parallèlement à l'axe.

4. Dispositif de visée selon la revendication 3, caractérisé en ce que la fente de serrage (16) ne s'étend que sur une partie de la section transversale du bras de visée.

5. Combinaison d'un dispositf de visée selon la revendication 4 avec une douille, caractérisée en ce qu'une douillle (17) pouvant être enfilée sur le bras de visée (9) dans la région des trous de visée (10 à 15) présente une première section de paroi (18) qui peut être verrouillée par précontrainte élastique avec une deuxième section de paroi (20), la première section de paroi comprimant l'une contre l'autre les régions de la section transversale du bras de visée séparées par la fente de serrage (16),
et en ce que la douille (17) présente des trous de passage correspondant aux trous de visée (10 à 15).

6. Combinaison selon la revendication 5, caractérisée en ce que le bras de visée (9) a une section à peu près rectangulaire et que la douille (17) a une section en caisson correspondante, en ce que la première et la deuxième section de paroi sont deux parois de douille (18, 20) orientées perpendiculairement l'une à l'autre, en ce que la première section de paroi (18) est incurvée, en partant de son côté assemblé rigidement à une autre paroi de douille (19), en s'écartant du centre de la douille (17) et peut être verrouillée par son bord libre sous une saillie (19) de la paroi de douille (20) séparée de ce bord, de la deuxième section de paroi.

7. Combinaison selon l'une des revendications 6 ou 7, caractérisée en ce qu'il est prévu plusieurs douilles (17) pouvant être insérées sur le bras de visée (9) dans la région des trous de visée (10 à 15) et pouvant être fixées à celui-ci, ces douilles ayant chacune un seul trou de passage pour l'un des différents trous de visée (10 à 13) du bras de visée pour des trous obliques (27) et, éventuellement, un trou de passage pour un trou de visée (14, 15) du bras de visée pour un trou transversal (32).

8. Dispositif de visée selon l'une des revendications 1 à 4, caractérisé en ce que la tête (2) et le bras de visée (9) sont assemblés l'un à l'autre de manière indissociable par une pièce de maintien (8), coudée, transparente aux rayons X.

9. Dispositif de visée selon l'une des revendications 1 à 4 ou 8, caractérisé en ce que la tête (2) et le bras de visée (9) sont constitués de métal léger et, de préférence, d'un alliage d'aluminium ou de titane.

10. Dispositif de visée selon l'une des revendications 1 à 4, 8 ou 9, caractérisé en ce que la tête présente, à peu près dans le plan formé par l'axe de la broche (3) et le bras de visée (9), deux trous de guidage (6) à peu près parallèles et approximativement perpendiculaires à l'axe de la broche, destinés à recevoir des fils de Kirschner latéralement en saillie pour un contrôle radiographique de position.
